# EUROPEAN PATENT APPLICATION

(11) **EP 4 353 820 A1**
(43) Date of publication of application: **17.04.2024**
(21) Application number: 22820531.6
(22) Date of filing: 07.06.2022
(51) Int. Cl.: C12N 9/02, C12N 15/81, C12P 21/02

(54) **SUPEROXIDE DISMUTASE 1 VARIANT AND METHOD FOR PRODUCING GLUTATHIONE OR DERIVATIVE THEREOF, USING SAME**

(30) Priority: 10.06.2021 KR 20210075689
(71) Applicant: CJ Cheiljedang Corporation, Seoul 04560 (KR)
(72) Inventor: IM, Yeong Eun, Seoul 04560 (KR); HA, Cheol Woong, Seoul 04560 (KR); YANG, Eun Bin, Seoul 04560 (KR); KIM, Yeonsoo, Seoul 04560 (KR)
(74) Representative: Regimbeau
(86) International application number: PCT/KR2022/008016
(87) International publication number: WO 2022/260403

(57) **Abstract**

The present application relates to a superoxide dismutase 1 variant and a method for producing glutathione or derivatives thereof using the same.

## Description

### [Technical Field]

The present application relates to a superoxide dismutase 1 variant and a method for producing glutathione or derivatives thereof using the same.

### [Background Art]

Glutathione (GSH) is an organosulfur compound most commonly found in cells, and it exists in the form of a tripeptide, in which three amino acids (i.e., glycine, glutamate, and cysteine) are conjugated.

In the body, glutathione exists in two forms: reduced glutathione (GSH) and oxidized glutathione (GSSG). Reduced glutathione is generally present in a relatively high percentage. It is mainly dispersed in the liver and skin cells of the human body, and has an important role in antioxidant functions, such as decomposing and removing oxygen free radicals, detoxification functions, such as removing foreign compounds (*i*.*e*., toxic substances), and whitening functions that inhibit the production of melanin, *etc.*

Because of these various functions, glutathione has attracted much attention as materials in various fields such as pharmaceuticals, health functional foods, cosmetics, *etc.,* and is also used in the preparation of flavoring ingredients, food, and feed additives. It is known that glutathione has a significant effect of increasing the flavor of raw materials and maintaining rich flavor, and can be used alone as *kokumi* flavor enhancer or in combination with other substances. Generally, *kokumi* material has a richer flavor than *umami* material, such as nucleic acid and MSG, and is known to be produced by decomposition and aging of proteins. Further, glutathione may also be converted to other *gamma*-glutamyl peptides (Sofyanovich, O. A. et al., (2019) Multiple pathways for the formation of the γ-glutamyl peptides γ-glutamyl-valine and γ-glutamyl-valyl glycine in Saccharomyces cerevisiae. PLoS ONE 14 (5): e0216622). *gamma*-Glutamyl peptide is a low-molecular-weight compound having a *gamma-*carboxyl group of glutamic acid at the N-terminus of the molecule, and is known to be widely used as a *kokumi* flavor enhancer.

However, despite the increased demand for glutathione and derivatives thereof that can be used in various fields as described above, the enzyme synthesis process has not yet been commercialized due to high production cost, and accordingly, the market has not been significantly activated due to the considerable cost required for the industrial production of glutathione and its derivatives.

### [Disclosure]

### [Technical Problem]

In order to solve the problems, the present application provides a superoxide dismutase 1 variant and a method for producing glutathione or derivatives thereof using the same.

### [Technical Solution]

It is one object of the present application to provide a superoxide dismutase 1 variant, in which an amino acid corresponding to the position 37 from the N-terminus of the amino acid sequence of SEQ ID NO: 1 is substituted with a different amino acid.

It is another object of the present application to provide a polynucleotide encoding the variant, and a vector including the polynucleotide.

It is still another object of the present application to provide a microorganism including any one or more of the variant and a polynucleotide encoding the variant.

It is yet another object of the present application to provide a method for producing glutathione or derivatives thereof, including culturing the microorganism.

### [Advantageous Effects]

The novel superoxide dismutase 1 variant of the present application can be effectively used in high production of glutathione or derivatives thereof.

### [Brief Description of Drawing]

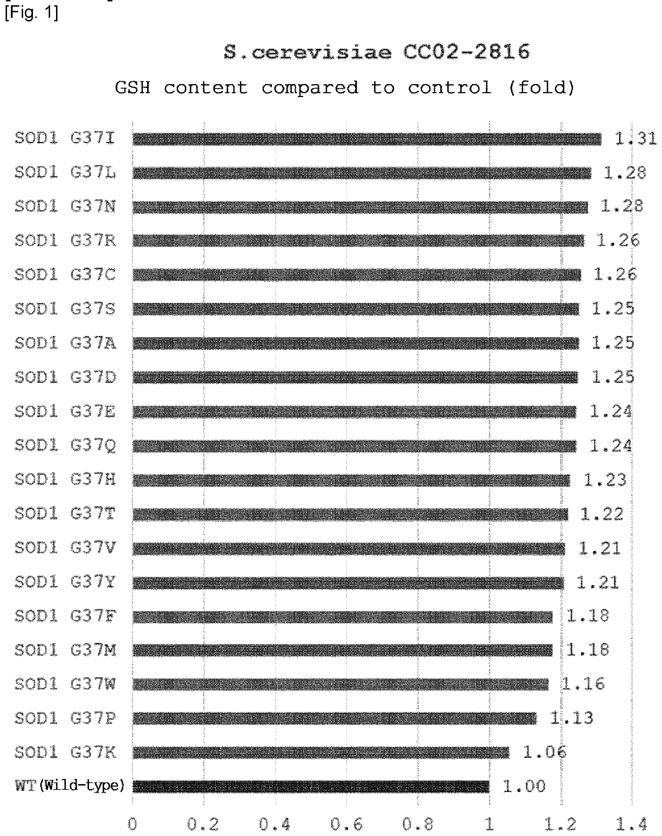

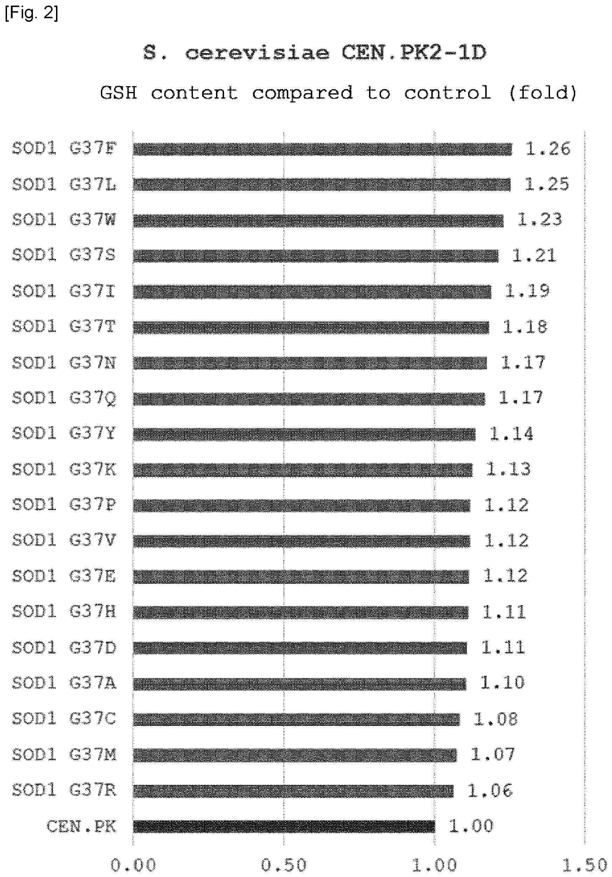

Figs. 1 and 2 show the result of confirming the glutathione production level by introducing the superoxide dismutase 1 variant into a strain of *Saccharomyces cerevisiae.*

### [Detailed Description of Preferred Embodiments]

The present application will be described in detail as follows. Meanwhile, each description and embodiment disclosed herein can be applied to other descriptions and embodiments, respectively. That is, all combinations of various elements disclosed herein fall within the scope of the present application. Further, the scope of the present application is not limited by the specific description described below.

Additionally, those of ordinary skill in the art may be able to recognize or confirm, using only conventional experimentation, many equivalents to the particular aspects of the invention described herein. Furthermore, it is also intended that these equivalents be included in the present application.

In addition, a number of papers and patent documents have been referenced and cited throughout the present specification. The content of the cited papers and patent documents is incorporated herein by reference in their entirety, and the level of technical field to which the present invention belongs and the contents of the present invention will be described more clearly.

One aspect of the present application provides a superoxide dismutase 1 variant, including an amino acid substitution in the protein having superoxide dismutase 1 (SOD1) activity, wherein the substitution includes substituting the amino acid corresponding to the position 37 from the N-terminus of SEQ ID NO: 1 with a different amino acid.

The variant may be a protein variant in which the amino acid corresponding to the position 37 from the N-terminus in the amino acid sequence of SEQ ID NO: 1 is substituted with a different amino acid.

The "superoxide dismutase 1" of the present application is an enzyme also referred to as "SOD1" or "superoxide dismutase [Cu-Zn]". The superoxide dismutase is known to catalyze the following Reaction Scheme:

2O₂⁻ + 2H⁺ → H₂O₂ + O₂

As used herein, the amino acid sequence of superoxide dismutase 1 is an amino acid sequence encoded by the *sod1* gene, and may be referred to as "SOD1 protein". The amino acid constituting the superoxide dismutase 1 of the present application can be obtained from GenBank of NCBI, a known database. The superoxide dismutase 1 may be a protein including the amino acid sequence of SEQ ID NO: 1, but is not limited thereto. In another example, the superoxide dismutase 1 may be derived from *Saccharomyces cerevisiae.* In still another example, it may be one in which the amino acid corresponding to the position 37 in the amino acid sequence of SEQ ID NO: 1 is glycine, but is not limited thereto, and any sequence having the same superoxide dismutase 1 activity as the amino acid sequence above may be included without limitation.

In a specific example, the superoxide dismutase 1 of the present application may be a protein including the amino acid sequence of SEQ ID NO: 1 or an amino acid sequence having a homology or identity of 80%, 85%, 90%, 95%, 96%, 97%, 98%, or 99% or more thereto. Further, it is apparent that any protein having an amino acid sequence, in which part of the amino acid sequence is deleted, modified, substituted, or added, may also fall within the scope of the protein targeted for modification of the present application as long as it is an amino acid sequence having such a homology or identity and exhibiting an effect corresponding to that of the above protein.

Additionally, although in the present application, the superoxide dismutase 1 is defined as a protein including the amino acid sequence of SEQ ID NO: 1 in one example, it does not exclude a mutation that may occur by a meaningless sequence addition upstream or downstream of the amino acid sequence of SEQ ID NO: 1 or that may occur naturally, or a silent mutation thereof, and it is apparent to those skilled in the art that any protein having the same or corresponding activity to the protein composed of the amino acid sequence of SEQ ID NO: 1 may fall within the superoxide dismutase 1 of the present application.

That is, in the present application, although it is expressed as "a protein or polypeptide having an amino acid sequence of a particular SEQ ID NO" or "a protein or polypeptide including an amino acid sequence of a particular SEQ ID NO", it is apparent that any protein having an amino acid sequence, in which part of the amino acid sequence is deleted, modified, substituted, or added, may also be used in the present application, as long as the protein has the same or corresponding activity to the polypeptide composed of the amino acid sequence of the corresponding SEQ ID NO.

As used herein, the term "variant" or "modified polypeptide" refers to a protein having one or more amino acids different from the recited sequence by conservative substitutions and/or modifications such that the functions or properties of the protein are retained.

The variants are different from the sequences identified by substitution, deletion or addition of several amino acids. Such variants may generally be identified by modifying one or more of the above amino acid sequences of the protein and evaluating the properties of the modified protein. That is, the ability of the variants may be enhanced, unchanged or reduced relative to a native protein. Additionally, some variants may include modified polypeptides in which one or more regions, such as an N-terminal leader sequence or transmembrane domain, have been removed. Other variants may include those in which a region has been removed from the N- and/or C-terminus of a mature protein. The term "variant" or "modified polypeptide" may be used interchangeably with terms such as modification, modified protein, mutant, mutein, divergent, variant, *etc.,* as long as the terms are used to indicate variation, but the terms are not limited thereto.

As used herein, the term "conservative substitution" refers to substitution of an amino acid with another amino acid having similar structural and/or chemical properties. The variant may have, for example, one or more conservative substitutions while still retaining one or more biological activities. Such amino acid substitution may generally occur based on similarity of polarity, charge, solubility, hydrophobicity, hydrophilicity, and/or amphipathic nature of a residue.

For example, among the amino acids having an electrically charged side chain, positively charged (basic) amino acids include arginine, lysine, and histidine; negatively charged (acidic) amino acids include glutamic acid and aspartic acid; and amino acids having uncharged side chain include glycine, alanine, valine, leucine, isoleucine, methionine, phenylalanine, tryptophan, proline, serine, threonine, cysteine, tyrosine, asparagine, and glutamine.

Additionally, the variant may also include deletion or addition of amino acids that have minimal influence on the properties and secondary structure of a polypeptide. For example, the polypeptide may be conjugated with a signal (or leader) sequence at the N-terminus involved in the transfer of proteins co-translationally or post-translationally. Further, the polypeptide may also be conjugated with another sequence or linker to identify, purify, or synthesize the polypeptide.

In one embodiment, the variant of the present application may be a superoxide dismutase 1 variant, in which an amino acid corresponding to the position 37 from the N-terminus of the amino acid sequence of SEQ ID NO: 1 is substituted with a different amino acid, or a modified polypeptide having the superoxide dismutase 1 activity, among the superoxide dismutase 1 described above. The variant of the present application, *i*.*e*., the "superoxide dismutase 1 variant" may be expressed as "a (modified) polypeptide having superoxide dismutase 1 activity" or "SOD1 variant", or may be one that can increase glutathione production as compared to that of a natural wild-type polypeptide or a non-modified polypeptide, but is not limited thereto.

In any one of the above-described embodiments, the variant may be a superoxide dismutase 1 variant, in which an amino acid corresponding to the position 37 from the N-terminus of the amino acid sequence of SEQ ID NO: 1 is substituted with a different amino acid.

In the present application, the "substitution with a different amino acid" is not limited as long as it is an amino acid different from the amino acid before substitution. Meanwhile, in the present application, when it is expressed that "a specific amino acid has been substituted", it is apparent that the amino acid is substituted with an amino acid different from the amino acid before substitution, even if it is not specifically stated that the amino acid has been substituted with a different amino acid.

In any one of the above-described embodiments, the "different amino acid" may be an amino acid except glycine. Specifically, the different amino acid may be an amino acid selected from alanine, valine, leucine, isoleucine, methionine, phenylalanine, tryptophan, proline, serine, threonine, cysteine, tyrosine, asparagine, glutamine, aspartic acid, glutamic acid, lysine, arginine, and histidine, but is not limited thereto.

As used herein, the "corresponding position" refers to an amino acid residue at the position recited in a protein or peptide, or an amino acid residue which is similar, identical, or homologous to the residue recited in a protein or peptide. As used herein, the "corresponding region" generally refers to a similar or corresponding position in the related protein or reference protein.

In the present application, a specific numbering of amino acid residue positions in the protein used herein may be employed. For example, it is possible to renumber the amino acid residue positions of the protein of the present application to the corresponding positions by aligning the peptide sequence of the protein of the present application with the target protein to be compared.

The superoxide dismutase 1 variant, in which an amino acid corresponding to the position 37 from the N-terminus of the amino acid sequence of SEQ ID NO: 1 is substituted with a different amino acid, may be a protein in which an amino acid corresponding to the position 37 of SEQ ID NO: 1 is substituted with a different amino acid, among the amino acid sequence of SEQ ID NO: 1 or an amino acid sequence having a homology or identity of 80%, 85%, 90%, 95%, 96%, 97%, 98%, or 99% or more thereto.

Such a variant may be a variant having a homology or identity of 80%, 85%, 90%, 95%, 96%, 97%, 98%, or 99% or more to SEQ ID NO: 1, and having a homology or identity of less than 100% to SEQ ID NO: 1, but is not limited thereto.

The superoxide dismutase 1 variant, in which an amino acid corresponding to the position 37 from the N-terminus of the amino acid sequence of SEQ ID NO: 1 is substituted with a different amino acid, may include any one of amino acid sequences of SEQ ID NOS: 12 to 30. Specifically, the variant may consist essentially of any one of amino acid sequences of SEQ ID NOS: 12 to 30, and more specifically consist of any one of amino acid sequences of SEQ ID NOS: 12 to 30.

In addition, the variant may include any one of amino acid sequences of SEQ ID NOS: 12 to 30, or may include an amino acid sequence, in which the amino acid at position 37 in the amino acid sequence is fixed (i.e., in the amino acid sequence of the variant, the amino acid corresponding to the position 37 in the amino acid sequence of SEQ ID NOS: 12 to 30 is identical to the amino acid at position 37 in the amino acid sequence of SEQ ID NOS: 12 to 30) and which has a homology or identity of 80% or more thereto, but is not limited thereto.

Specifically, the variant of the present application may have any one of amino acid sequences of SEQ ID NOS: 12 to 30 and may have a homology or identity of at least 80%, 90%, 95%, 96%, 97%, 98%, or 99% to the amino acid sequences of any one of amino acid sequences of SEQ ID NOS: 12 to 30, wherein the amino acid corresponding to the position 37 in the amino acid sequence of any one of SEQ ID NOS: 12 to 30 may include a polypeptide, which is an amino acid other than glycine. Additionally, it is apparent that any protein having an amino acid sequence, in which part of the amino acid sequence is deleted, modified, substituted, or added, may fall within the scope of the present application, as long as the amino acid sequence has such homology or identity and shows an efficacy corresponding to that of the variant.

As used herein, the term "homology" or "identity" refers to a degree of relatedness between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and can be used with a default gap penalty established by the program being used. Substantially, homologous or identical sequences may hybridize under moderately or highly stringent conditions such that the full length of the sequence or at least about 50%, 60%, 70%, 80%, or 90% or more of the full-length may hybridize. It is apparent that hybridization with polynucleotides containing general codons or degenerate codons is also included.

Whether any two polynucleotide or polypeptide sequences have a homology, similarity, or identity may be, for example, determined by a known computer algorithm such as the "FASTA" program (Pearson et al., (1988) Proc. Natl. Acad. Sci. USA 85:2444) using default parameters. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (preferably, version 5.0.0 or later) (GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST or ClustalW of the National Center for Biotechnology Information (NCBI).

The homology, similarity, or identity of polynucleotides or polypeptides may be, for example, determined by comparing sequence information using, for example, the GAP computer program, such as Needleman et al. (1970), J Mol Biol. 48:443 as disclosed in Smith and Waterman, Adv. Appl. Math (1981) 2:482. In summary, the GAP program defines the homology, similarity, or identity as the value obtained by dividing the number of similarly aligned symbols (i.e., nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have a homology, similarity or identity with each other may be identified by comparing the sequences in a Southern hybridization experiment under stringent conditions as defined, and appropriate hybridization conditions defined are within the skill of the art, and may be determined by a method well known to those skilled in the art (*e*.*g*., J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York).

Another aspect of the present application may provide a polynucleotide encoding the variant.

As used herein, the term "polynucleotide", which is a polymer of nucleotides composed of nucleotide monomers connected in a lengthy chain by a covalent bond, is a DNA or RNA strand having at least a certain length.

The gene encoding the superoxide dismutase 1 of the present application may be the *sod1* gene.

The gene may be derived from yeast. Specifically, it may be derived from the genus *Saccharomyces,* more specifically from *Saccharomyces cerevisiae.* Specifically, any gene may be included without limitation as long as it encodes a polypeptide having the activity of superoxide dismutase 1 derived from *Saccharomyces cerevisiae.* In one embodiment, it may be a gene encoding the amino acid sequence of SEQ ID NO: 1, and in another embodiment, it may include a nucleotide sequence of SEQ ID NO: 2 or SEQ ID NO: 3, but is not limited thereto.

The polynucleotide encoding the superoxide dismutase 1 variant of the present application may include any polynucleotide encoding the superoxide dismutase 1 variant or a polypeptide having an activity corresponding thereto without limitation.

The superoxide dismutase 1 of the present application or the polynucleotide encoding a variant thereof may undergo various modifications in the coding region within the scope that does not change the amino acid sequence of the polypeptide, due to codon degeneracy or in consideration of the codons preferred in an organism in which the polypeptide is to be expressed.

Specifically, the polynucleotide encoding the superoxide dismutase 1 variant of the present application may include any polynucleotide sequence without limitation as long as it encodes a protein variant, in which the amino acid corresponding to the position 37 in the amino acid sequence of SEQ ID NO: 1 with a different amino acid.

For example, the polynucleotide encoding the protein variant of the present application may be the protein variant of the present application, specifically a polynucleotide sequence encoding any one of amino acid sequences of SEQ ID NOS: 12 to 30 or a polypeptide having a homology or identity thereto, and the sequence encoding the amino acid corresponding to the position 37 in any one amino acid sequences of SEQ ID NOS: 12 to 30 may be a polynucleotide sequence encoding an amino acid except glycine, but is not limited thereto. The homology and identity are as described above.

Further, the polynucleotide encoding the protein variant of the present application may include a probe that may be prepared from a known gene sequence, for example, any sequence encoding the protein variant in which the amino acid corresponding to the position 37 in the amino acid sequence of SEQ ID NO: 1 is substituted with a different amino acid, which can hybridize with a sequence complementary to all or part of the nucleotide sequence under stringent conditions without limitation.

The "stringent conditions" refers to conditions under which specific hybridization between polynucleotides is allowed. Such conditions are specifically described in the literature (*e*.*g*., J. Sambrook *et al*.). For example, the stringent conditions may include conditions under which genes having a high homology or identity of 40% or more, specifically 90% or more, more specifically 95% or more, 96% or more, 97% or more, 98% or more, or even more specifically 99% or more, are hybridized with each other and genes having a homology or identity lower than the above homologies or identities are not hybridized with each other, or washing conditions of Southern hybridization, i.e., washing once, specifically, twice or three times at a salt concentration and a temperature corresponding to 60°C, 1×SSC, 0.1% SDS, specifically 60°C, 0.1×SSC, 0.1% SDS, and more specifically 68°C, 0.1×SSC, 0.1% SDS.

Hybridization requires that two nucleic acids contain complementary sequences, although mismatches between bases are possible depending on the stringency of the hybridization. The term "complementary" is used to describe the relationship between nucleotide bases that can hybridize with each other. For example, with respect to DNA, adenine is complementary to thymine, and cytosine is complementary to guanine. Therefore, the polynucleotide of the present application may include isolated nucleic acid fragments complementary to the entire sequence as well as nucleic acid sequences substantially similar thereto.

Specifically, polynucleotides having a homology or identity may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (*e*.*g*., Sambrook *et al*., *supra*, 9.50-9.51, 11.7-11.8).

Still another aspect of the present application may provide a vector including a polynucleotide encoding the protein variant.

As used herein, the term "vector" refers to a DNA construct containing the nucleotide sequence of a polynucleotide encoding the target protein operably linked to a suitable expression regulatory region (or expression regulatory sequence) so as to be able to express the target polypeptide in a suitable host cell. The expression regulatory region may include a promoter capable of initiating transcription, any operator sequence for regulating the transcription, a sequence encoding a suitable mRNA ribosome binding site, and a sequence for regulating termination of transcription and translation. Once transformed into a suitable host cell, the vector may replicate or function independently from the host genome, or may integrate into genome thereof.

In one example, a polynucleotide encoding a target protein in the chromosome may be replaced with a modified polynucleotide through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface proteins, may be used. Only cells expressing the selection marker are able to survive or to show different phenotypes under the environment treated with the selective agent, and thus the transformed cells may be selected.

The vector used in the present application is not particularly limited, and any vector known in the art may be used. Yeast expression vector may include both yeast integrative plasmid (YIp) and extrachromosomal plasmid vector. The extrachromosomal plasmid vector may include yeast episomal plasmid (Yep), yeast replicative plasmids (YRp), and yeast centromere plasmid (YCp). In addition, yeast artificial chromosomes (YACs) can also be used as the vector of the present application. Specific examples of the vector that can be used may include pESCHIS, pESC-LEU, pESC-TRP, pESC-URA, Gateway pYES-DEST52, pAO815, pGAPZ A, pGAPZ B, pGAPZ C, pGAPα A, pGAPα B, pGAPα C, pPIC3.5K, pPIC6 A, pPIC6 B, pPIC6 C, pPIC6α A, pPIC6α B, pPIC6α C, pPIC9K, pYC2/CT, pYD1 Yeast Display Vector, pYES2, pYES2/CT, pYES2/NT A, pYES2/NT B, pYES2/NT C, pYES2/CT, pYES2.1, pYES-DEST52, pTEF1/Zeo, pFLD1, PichiaPinkTM, p427-TEF, p417-CYC, pGAL-MF, p427-TEF, p417-CYC, PTEF-MF, pBY011, pSGP47, pSGP46, pSGP36, pSGP40, ZM552, pAG303GAL-ccdB, pAG414GAL-ccdB, pAS404, pBridge, pGAD-GH, pGAD T7, pGBK T7, pHIS-2, pOBD2, pRS408, pRS410, pRS418, pRS420, pRS428, yeast micron A form, pRS403, pRS404, pRS405, pRS406, pYJ403, pYJ404, pYJ405, and pYJ406, but are not limited thereto.

As used herein, the term "transformation" refers to the introduction of a vector including a polynucleotide encoding a target protein into a host cell so that the protein encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. Further, the polynucleotide may include DNA and RNA encoding the target protein. The polynucleotide may be introduced in any form, as long as it can be introduced into the host cell and expressed therein. For example, the polynucleotide may be introduced into the host cell in the form of an expression cassette, which is a gene construct including all elements required for its autonomous expression. The expression cassette may commonly include a promoter operably linked to the polynucleotide, a transcription terminator, a ribosome binding site, or a translation terminator. The expression cassette may be in the form of a self-replicable expression vector. Additionally, the polynucleotide may be introduced into the host cell as it is and operably linked to sequences required for expression in the host cell, but is not limited thereto.

Further, as used herein, the term "operably linked" means that the gene sequence is functionally linked to a promoter sequence that initiates and mediates transcription of the polynucleotide encoding the target polypeptide of the present application.

The method for transforming the vector of the present application may include any method which can introduce a nucleic acid into a cell, and the transformation may be performed by selecting an appropriate standard technique as known in the art according to the host cell. For example, the method may include electroporation, calcium phosphate (CaPO₄,) precipitation, calcium chloride (CaCl₂) precipitation, microinjection, a polyethylene glycol (PEG) method, a DEAE-dextran method, a cationic liposome method, and a lithium acetate-DMSO method, *etc.,* but is not limited thereto.

The present application may provide a microorganism including any one or more of the superoxide dismutase 1 and a polynucleotide encoding the variant.

As used herein, the term "microorganism" includes all wild-type microorganisms, or genetically modified microorganisms by natural or artificial means, and is a comprehensive concept including all microorganisms in which a particular mechanism is weakened or enhanced due to insertion of a foreign gene, or enhancement or weakening of the activity of an endogenous gene.

In one embodiment, the microorganism may be a microorganism that produces glutathione. As used herein, the term "microorganism that produces glutathione" may be used interchangeably with terms such as "glutathione-producing microorganism", "microorganism having glutathione producing ability", "glutathione-producing strain", "strain having glutathione producing ability", *etc.*

In any one of the above-described embodiments, the microorganism may be a microorganism that produces a glutathione derivative.

In any one of the above-described embodiments, the microorganism of the present application may include a vector including the polynucleotide encoding the superoxide dismutase 1.

The "glutathione" of the present application may be used interchangeably with "glutathione" or "GSH", and refers to a tripeptide composed three amino acids (i.e., glutamate, cysteine, glycine). Glutathione may be used as a raw material for pharmaceuticals, health functional foods, flavoring materials, food, feed additives, cosmetics, *etc.,* but is not limited thereto.

The "glutathione derivative" of the present application may include any substance that can be converted using glutathione as a starting material without limitation.

In the present application, glutathione or derivatives thereof may be collectively referred to as "*gamma*-glutamyl peptide". *gamma*-Glutamyl peptide refers to a low-molecular-weight compound having a *gamma*-carboxyl group of glutamic acid at the N-terminus of the molecule.

Specifically, the *gamma*-glutamyl peptide may be a compound represented by General Formula 1 below:

[General Formula 1] γ-Glu -X-Y

In General Formula above, X is cysteine, glutamic acid, tyrosine, methionine, valine, tryptophan, aspartic acid, isoleucine, leucine, asparagine, glutamine, histidine, lysine, ornithine, arginine, *S*-methylcysteine, or *S*-ethylcysteine;

Y is glycine, alanine, leucine, isoleucine, serine, threonine, methionine, cysteine, aspartic acid, asparagine, glutamine, lysine, ornithine, arginine, phenylalanine, tyrosine, proline, hydroxyproline, *alpha*-aminobutyric acid, or absent.

In one embodiment, the gamma-glutamyl peptide may be selected from γ-Glu-Cys-Gly (glutathione), γ-Glu-Glu (γ-EF), γ-Glu-Tyr (γ-EY), γ-Glu-Met (γ-EM), γ-Glu-Val (γ-EV), γ-Glu-Trp (γ-EW), γ-Glu-Asp (γ-ED), γ-Glu-Ile (γ-EI), γ-Glu-Leu (γ-EL), γ-Glu-Cys-Gly (γ-ECG), γ-Glu-Val-Gly (γ-EVG), γ-glutamyl-S-methyl-cysteine (γ-E-S-methyl-C), γ-glutamyl-S-ethenyl-cysteine (γ-E-S-ethenyl-C), and γ-glutamyl-cysteinyl-β-alanine (γ-E-C-β-A).

In any one of the above-described embodiments, the gamma-glutamyl peptide may be glutathione, but is not limited thereto.

The microorganism of the present application may refer to a microorganism capable of producing an excess of desired glutathione, including superoxide dismutase 1, compared to a wild-type or unmodified microorganism. The microorganism is not particularly limited by its type as long as it can produce glutathione, but may be a microorganism of the genus *Saccharomyces,* specifically *Saccharomyces cerevisiae.* However, the microorganism is not limited thereto.

The parent strain of the glutathione-producing microorganism including the variant is not particularly limited as long as it is capable of producing glutathione. The microorganism may further include mutations such as strengthening biosynthetic pathways for increasing glutathione producing ability, releasing feedback inhibition, or gene inactivation that weakens degradation pathways or biosynthetic pathways, and these mutations do not exclude those that are naturally occurring.

As used herein, the term "enhancement" of a polypeptide activity means that the activity of a polypeptide is increased compared to its endogenous activity. The enhancement may be used interchangeably with terms such as activation, up-regulation, overexpression, increase, *etc.* In particular, the activation, enhancement, up-regulation, overexpression and increase may include both cases in which an activity not originally possessed is exhibited, or the activity is enhanced compared to the endogenous activity or the activity before modification. The "endogenous activity" refers to the activity of a particular polypeptide originally possessed by a parent strain before transformation or a non-modified microorganism, when a trait is altered through genetic modification caused by natural or artificial factors, and may be used interchangeably with "activity before modification". The "enhancement", "up-regulation", "overexpression", or "increase" in the activity of a polypeptide compared to its endogenous activity means that the activity and/or concentration (expression level) of the polypeptide is enhanced compared to that of a particular polypeptide originally possessed by a parent strain before transformation or a non-modified microorganism.

The enhancement may be achieved by introducing a foreign polypeptide, or by enhancing the activity and/or concentration (expression level) of the endogenous polypeptide. The enhancement of the activity of the polypeptide can be confirmed by the increase in the level of activity of the polypeptide, expression level, or the amount of product excreted from the polypeptide.

The enhancement of the activity of the polypeptide can be applied by various methods well known in the art, and is not limited as long as it can enhance the activity of the target polypeptide compared to that of the microorganism before modification. Specifically, genetic engineering and/or protein engineering well known to those skilled in the art, which is a common method of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, *etc*.).

Specifically, the enhancement of the polypeptide of the present application may be achieved by:
1) increasing the intracellular copy number of a polynucleotide encoding the polypeptide;
2) replacing the expression regulatory region of a gene encoding the polypeptide on the chromosome with a sequence having a strong activity;
3) modifying a nucleotide sequence encoding the initiation codon or 5'-UTR of the gene transcript encoding the polypeptide;
4) modifying the amino acid sequence of the polypeptide such that the activity of the polypeptide is enhanced;
5) modifying the polynucleotide sequence encoding the polypeptide such that the activity of the polypeptide is enhanced (*e*.*g*., modifying the polynucleotide sequence of the polypeptide gene to encode a polypeptide that has been modified to enhance the activity of the polypeptide);
6) introducing a foreign polypeptide exhibiting the polypeptide activity or a foreign polynucleotide encoding the same;
7) codon-optimization of the polynucleotide encoding the polypeptide;
8) analyzing the tertiary structure of the polypeptide and thereby selecting and modifying the exposed site, or chemically modifying the same; or
9) a combination of two or more selected from above 1 to 8), but is not particularly limited thereto.

More specifically,
the 1) method of increasing the intracellular copy number of a polynucleotide encoding the polypeptide may be achieved by introducing a vector, which is operably linked to the polynucleotide encoding the polypeptide and is able to replicate and function regardless of a host cell, into the host cell. Alternatively, the method may be achieved by introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto. The vector is as described above.

The 2) method of replacing the expression regulatory region (or expression regulatory sequence) of a gene encoding the polypeptide on the chromosome with a sequence having a strong activity may be achieved by inducing a modification on the sequence through deletion, insertion, non-conservative or conservative substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome binding site, and a sequence regulating the termination of transcription and translation, *etc.* In one example, the method may include replacing the original promoter with a strong promoter, but is not limited thereto.

Examples of promoters known for eukaryotes include promoters for translation elongation factor 1 (TEF1), glycerol-3-phosphate dehydrogenase 1 (GPD1), 3-phosphoglycerate kinase, or other glycolytic enzymes, for example, enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triose phosphate isomerase, phosphoglucose isomerase, and glucokinase; examples of other yeast promoters, which are inducible promoters with the additional advantage of controlled transcription by growth conditions include promoters for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization; promoters that can be used when the host cell is yeast may include TEF1 promoter, TEF2 promoter, GAL10 promoter, GAL1 promoter, ADH1 promoter, ADH2 promoter, PHO5 promoter, GAL1-10 promoter, TDH3 promoter(GPD promoter), TDH2 promoter, TDH1 promoter, PGK1 promoter, PYK2 promoter, ENO1 promoter, ENO2 promoter, and TPI1 promoter; and suitable vectors and promoters for use in yeast expression are further described in European Patent No. 073657, but the promoters are not limited thereto. In addition, yeast enhancers may also be advantageously used in combination with yeast promoters, but are not limited thereto.

The 3) method of modifying a nucleotide sequence encoding the initiation codon or 5'-UTR of the gene transcript encoding the polypeptide may be achieved, for example, by substituting the nucleotide sequence with a nucleotide sequence encoding another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or by replacing the sequence with an amino acid sequence or polynucleotide sequence modified to have a stronger activity, or an amino acid sequence or polynucleotide sequence modified to enhance the activity, but are not limited thereto. The replacement may specifically be performed by inserting the polynucleotide into the chromosome by homologous recombination, but is not limited thereto. The vector used herein may further include a selection marker to confirm the insertion into the chromosome. The selection marker is as described above.

The 6) method of introducing a foreign polynucleotide exhibiting the activity of the polypeptide may be achieved by introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables to produce the polypeptide, thereby increasing its activity.

The 7) method of codon-optimization of the polynucleotide encoding the polypeptide may be achieved by codon-optimization of an endogenous polynucleotide to increase the transcription or translation within a host cell, or by optimizing the codons thereof such that the optimized transcription and translation of the foreign polynucleotide can be achieved within the host cell.

The 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

Such enhancement of the polypeptide activity may mean that the activity or concentration of the corresponding polypeptide is increased relative to the activity or concentration of the polypeptide expressed in a wild-type or a microorganism before modification, or that the amount of product produced from the polypeptide is increased, but is not limited thereto.

The modification of a part or all of the polynucleotide in the microorganism of the present application may be achieved by (a) homologous recombination using a vector for chromosomal insertion in the microorganism or genome editing using engineered nuclease (*e*.*g*., CRISPR-Cas9) and/or (b) may be induced by light, such as ultraviolet rays and radiation, *etc.,* and/or chemical treatments, but is not limited thereto. The method of modifying a part or all of the gene may include a method by DNA recombination technology. For example, a part or all of the gene may be deleted by injecting a nucleotide sequence or a vector containing a nucleotide sequence homologous to the target gene into a microorganism to induce homologous recombination. The injected nucleotide sequence or the vector may include a dominant selection marker, but is not limited thereto.

As used herein, the term "weakening" of a polypeptide is a comprehensive concept including both reduced or no activity compared to its endogenous activity. The weakening may be used interchangeably with terms such as inactivation, deficiency, down-regulation, decrease, reduce, attenuation, *etc.*

The weakening may also include a case where the polypeptide activity itself is decreased or removed compared to the activity of the polypeptide originally possessed by a microorganism due to a mutation of the polynucleotide encoding the polypeptide; a case where the overall level of intracellular polypeptide activity and/or concentration (expression level) is decreased compared to a natural strain due to the inhibition of expression of the gene of the polynucleotide encoding the polypeptide, or the inhibition of translation into the polypeptide, *etc.;* a case where the polynucleotide is not expressed at all; and/or a case where no polypeptide activity is observed even when the polynucleotide is expressed. As used herein, the term "endogenous activity" refers to the activity of a particular polypeptide originally possessed by a parent strain before transformation, a wild-type or a non-modified microorganism, when a trait is altered through genetic modification caused by natural or artificial factors, and may be used interchangeably with "activity before modification". The expression that the polypeptide activity is "inactivated, deficient, decreased, down-regulated, reduced or attenuated" compared to its endogenous activity means that the polypeptide activity is decreased compared to the activity of a particular polypeptide originally possessed by a parent strain before transformation or a non-modified microorganism.

The weakening of the polypeptide activity can be performed by any method known in the art, but the method is not limited thereto, and can be achieved by applying various methods well known in the art (e.g., Nakashima N. et al., Bacterial cellular engineering by genome editing and gene silencing. Int J Mol Sci. 2014;15(2):2773-2793, Sambrook et al. Molecular Cloning 2012, *etc*.).

Specifically, the weakening of the polypeptide activity of the present application may be achieved by:
1) deleting a part or all of the gene encoding the polypeptide;
2) modifying the expression regulatory region (expression regulatory sequence) such that the expression of the gene encoding the polypeptide is decreased;
3) modifying the amino acid sequence constituting the polypeptide such that the polypeptide activity is removed or weakened (*e*.*g*., deletion/substitution/addition of one or more amino acids on the amino acid sequence);
4) modifying the gene sequence encoding the polypeptide such that the polypeptide activity is removed or weakened (*e*.*g*., deletion/substitution/addition of one or more of nucleotides on the nucleotide sequence of the polypeptide gene to encode a polypeptide that has been modified to remove or weaken the activity of the polypeptide);
5) modifying the nucleotide sequence encoding the initiation codon or 5'-UTR of the gene transcript encoding the polypeptide;
6) introducing an antisense oligonucleotide (*e*.*g*., antisense RNA), which binds complementary to the gene transcript encoding the polypeptide;
7) adding a sequence complementary to the Shine-Dalgarno (SD) sequence on the front end of the SD sequence of the gene encoding the polypeptide to form a secondary structure, thereby inhibiting the ribosomal attachment;
8) a reverse transcription engineering (RTE), which adds a promoter, which is to be reversely transcribed, on the 3' terminus of the open reading frame (ORF) of the gene sequence encoding the polypeptide; or
9) a combination of two or more selected from the methods 1) to 8) above, but is not particularly limited thereto.

For example,
the 1) method of deleting a part or all of the gene encoding the polypeptide may be achieved by deleting all of the polynucleotide encoding the endogenous target polypeptide within the chromosome, or by replacing the polynucleotide with a polynucleotide having a partially deleted nucleotide, or with a marker gene.
The 2) method of modifying the expression regulatory region (expression regulatory sequence) may be achieved by inducing a modification on the expression regulatory region (expression regulatory sequence) through deletion, insertion, non-conservative substitution or conservative substitution, or a combination thereof; or by replacing the sequence with a sequence having a weaker activity. The expression regulatory region may include a promoter, an operator sequence, a sequence encoding a ribosome binding site, and a sequence for regulating the termination of transcription and translation, but is not limited thereto.
The 5) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a lower polypeptide expression rate than the endogenous initiation codon, but is not limited thereto.
The 3) and 4) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to weaken the activity of the polypeptide, or by replacing the sequence with an amino acid sequence or a polynucleotide sequence modified to have a weaker activity, or an amino acid sequence or a polynucleotide sequence modified to have no activity, but are not limited thereto. For example, the expression of the gene may be inhibited or weakened by introducing a mutation into the polynucleotide sequence to form a termination codon, but is not limited thereto.
The 6) method of introducing an antisense oligonucleotide (e.g., antisense RNA), which binds complementary to the gene transcript encoding the polypeptide can be found in the literature (Weintraub, H. et al., Antisense-RNA as a molecular tool for genetic analysis, Reviews - Trends in Genetics, Vol. 1(1) 1986).
The 7) method of adding a sequence complementary to the Shine-Dalgarno (SD) sequence on the front end of the SD sequence of the gene encoding the polypeptide to form a secondary structure, thereby inhibiting the ribosome attachment may be achieved by inhibiting mRNA translation or reducing the speed thereof.
The 8) reverse transcription engineering (RTE), which adds a promoter, which is to be reversely transcribed, on the 3' terminus of the open reading frame (ORF) of the gene sequence encoding the polypeptide may be achieved by forming an antisense nucleotide complementary to the gene transcript encoding the polypeptide to weaken the activity.

In any one of the above-described embodiments, the microorganism of the present application may include a mutation in the expression regulatory region of glutamate-cysteine ligase (GSH1) to increase glutathione producing ability. The mutation may be any one or more mutations selected from -250(C→T), -252(G→A), -398(A→T), -399(A→C), -407(T→C), and -409(T→C) upstream of GSH1 ORF.

In any one of the above-described embodiments, the microorganism may include a mutation in which the 86^{th} amino acid and/or the 653^{rd} amino acid of glutamate-cysteine ligase (GSH1) are substituted with different amino acids. Specifically, the microorganism may include a mutation in which the 86^{th} amino acid is substituted with arginine and the 653^{rd} amino acid is substituted with methionine, but the mutation is not limited thereto.

In any one of the above-described embodiments, the microorganism may include a mutation in which the activity of glutamate-cysteine ligase (GSH1) is enhanced, but is not limited thereto.

The microorganism including any one or more of the superoxide dismutase 1 variant of the present application and a polynucleotide encoding the variant may be "a microorganism expressing the superoxide dismutase 1, in which the amino acid corresponding to the position 37 in the amino acid sequence of SEQ ID NO: 1 is substituted with a different amino acid", but is not limited thereto.

The superoxide dismutase 1 and the variants thereof are as described above.

As used herein, the term "to be expressed/expressing" with regard to a protein refers to a state in which a target protein is introduced into a microorganism or a target protein is modified to be expressed in a microorganism.

The microorganism expressing the protein variant of the present application may be a microorganism which has been modified to express the protein variant of the present application, and accordingly, yet another aspect of the present application provides a method for producing a microorganism expressing the protein variant of the present application.

As used herein, the term "strain before modification" or "microorganism before modification" does not exclude a strain containing a mutation that may occur naturally in a microorganism, and may refer to a wild-type strain or a natural strain itself, or a strain before the trait is altered due to genetic modification caused by natural or artificial factors. The "strain before modification" or "microorganism before modification" may be used interchangeably with "non-mutant strain", "non-modified strain", "non-mutant microorganism", "non-modified microorganism", or "reference microorganism".

In the present application, the microorganism including the superoxide dismutase 1 variant of the present application, a polynucleotide encoding the variant, or a vector including the polynucleotide may be a recombinant microorganism, and the recombination may be achieved by genetic modification such as transformation.

For example, the microorganism may be a recombinant microorganism prepared by transformation with a vector including the polynucleotide, but is not limited thereto. The recombinant microorganism may be yeast, e.g., a microorganism of the genus *Saccharomyces,* specifically *Saccharomyces cerevisiae,* but is not limited thereto.

Even another aspect of the present application provides a method for producing glutathione or derivatives thereof, including: culturing the microorganism. The microorganism, glutathione, or derivatives thereof are as described above.

The medium and other culture conditions used for culturing the strain of the present application may be any medium used for conventional cultivation of microorganisms of the genus *Saccharomyces* without any particular limitation. Specifically, the strain of the present application may be cultured under aerobic or anaerobic conditions in a conventional medium containing an appropriate carbon source, nitrogen source, phosphorus source, inorganic compound, amino acid, and/or vitamin, while adjusting temperature, pH, *etc.*

In the present application, the carbon source may include carbohydrates, such as glucose, fructose, sucrose, maltose, *etc.;* sugar alcohols, such as mannitol, sorbitol, *etc.;* organic acids, such as pyruvic acid, lactic acid, citric acid, *etc.;* amino acids, such as glutamate, methionine, lysine, *etc.,* but is not limited thereto. Additionally, the carbon source may include natural organic nutrients such as starch hydrolysate, molasses, blackstrap molasses, rice bran, cassava, sugar cane molasses, and corn steep liquor, *etc.,* and carbohydrates such as glucose and sterilized pretreated molasses (i.e., molasses converted to reducing sugar) may be used. In addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* and organic nitrogen sources, such as amino acids, peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compound may include sodium chloride, calcium chloride, iron chloride, magnesium sulfate, iron sulfate, manganese sulfate, calcium carbonate, *etc.*

Additionally, amino acids, vitamins, and/or appropriate precursors may be included in the medium. Specifically, L-amino acids, *etc.,* may be added to the culture medium of the strain. Specifically, glycine, glutamate, and/or cysteine may be added, and L-amino acids, such as lysine, may be further added when needed, but the present application is not limited thereto.

The medium or precursor may be added to the culture product in a batch or continuous manner, but is not limited thereto.

In the present application, the pH of a culture product may be adjusted during the cultivation of the strain by adding a compound such as ammonium hydroxide, potassium hydroxide, ammonia, phosphoric acid, sulfuric acid, *etc.* to the culture product in an appropriate manner. Additionally, during the cultivation, an antifoaming agent such as fatty acid polyglycol ester may be added to prevent foam generation. In addition, oxygen or oxygen-containing gas may be injected into the culture product in order to maintain an aerobic state of the culture product; or nitrogen, hydrogen, or carbon dioxide gas may be injected, or no gas may be injected, in order to maintain an anaerobic or microaerobic state of the medium.

The temperature of the culture product may be in a range from 25°C to 40°C, and specifically, from 28°C to 37°C, but is not limited thereto. The cultivation may be continued until a desired production amount of useful materials is obtained, specifically for 1 to 100 hours, but is not limited thereto.

The production method may further include an additional process after the culturing step. The additional process may be appropriately selected depending on the use of glutathione or derivatives thereof.

Specifically, the method for producing glutathione or derivatives thereof may include a step of recovering glutathione or derivatives thereof accumulated in cells by the culturing step, for example, the method may include a step of recovering glutathione or derivatives thereof from at least one material selected from the strains, a dried product thereof, an extract thereof, a culture product thereof, and a lysate thereof after the culturing step. In another example, the method for producing glutathione derivatives may include a step of converting glutathione into a derivative.

The method may further include a step of lysing the strain before the recovering step or simultaneously. The lysis of the strain may be performed by a method commonly used in the technical field to which the present application belongs, for example, by a lysis buffer solution, a sonicator, heat treatment, and a French press, *etc.* In addition, the lysis step may include, but is not limited to, enzymatic reactions such as cell wall lyases, nucleic acid lyases, nucleic acid transferases, and proteases, *etc.*

Dry yeast, yeast extract, yeast extract mix powder, pure glutathione or derivatives thereof containing a high content of glutathione or derivatives thereof may be prepared through the production method of the present application, but is not limited thereto, and may be appropriately prepared according to the desired product.

In the present application, the dry yeast may be used interchangeably with term such as "a dried product of the strain". The dry yeast may be prepared by drying yeast cells in which glutathione is accumulated, and may specifically be included in a composition for feed, a composition for food, *etc.,* but is not limited thereto.

As used herein, the yeast extract may be used interchangeably with term such as "an extract of the strain". The extract of the strain may mean a material remaining after separating the cell wall from the cells of the strain. Specifically, it may mean the remaining components excluding the cell wall from the components obtained by lysis of the cells. The extract of the strain includes glutathione or derivatives thereof, and other components may include one or more components of proteins, carbohydrates, nucleic acids, and fibers, but are not limited thereto.

In the recovering step, the target materials, i.e., glutathione or derivatives thereof, may be recovered by a suitable method known in the art.

The recovering step may include a purification process. The purification process may be pure purification by separation only of glutathione or derivatives thereof from the strain. Pure glutathione may be prepared from the purification process.

If necessary, the method for producing glutathione may further include a step of mixing a material selected from among the strains obtained after the culturing step, a dried product thereof, an extract thereof, a culture product thereof, a lysate thereof, and glutathione recovered therefrom with an excipient. Through the mixing step, yeast extract mix powder can be prepared. The same can be applied to derivatives of glutathione.

The excipient may be appropriately selected and used according to the intended use or form, for example, it may be selected from starch, glucose, cellulose, lactose, glycogen, D-mannitol, sorbitol, lactitol, maltodextrin, calcium carbonate, synthetic aluminum silicate, dibasic calcium phosphate, calcium sulfate, sodium chloride, sodium hydrogen carbonate, purified lanolin, dextrin, sodium alginate, methyl cellulose, colloidal silica gel, hydroxypropyl starch, hydroxypropyl methylcellulose, propylene glycol, casein, calcium lactate, Primojel^{®}, and Arabic gum, specifically one or more components selected from starch, glucose, cellulose, lactose, dextrin, glycogen, D-mannitol, and maltodextrin, but is not limited thereto.

The excipient may include, for example, a preservative, a wetting agent, a dispersing agent, a suspending agent, a buffer, a stabilizing agent, or an isotonic agent, but is not limited thereto.

### [Mode for Carrying Out the Invention]

The present application will be described in detail by way of Examples and Experimental Examples. However, these Examples and Experimental Examples are provided for illustrative purposes only, and the scope of the present application is not intended to be limited to or by these Examples and Experimental Examples.

### Example 1: Improvement of Glutathione-Producing Strain

In order to further improve the glutathione producing ability of the CJ-5 strain (KR 10-2222210 B1), which is a glutathione-producing strain deposited with Accession No. KCCM12568P, mutations were induced in the following manner.

The CJ-5 strain was cultured on a solid medium and then inoculated into the broth to obtain a culture solution, and UV was irradiated to the cells using a UV lamp. Thereafter, only the mutant strains that formed colonies by smearing the UV-irradiated culture solution on a plate medium were obtained by isolation, and the strain with the greatest improvement in glutathione producing ability was isolated.

As a result of confirming the genome of the isolated strain, mutations of -250(C→T), -252(G→A), -398(A→T), -399(A→C), -407(T→C), and -409(T→C) occurred in the upstream of the GSH1 ORF, and it was confirmed that cysteine, the amino acid at position 86 of the GSH1 protein encoded by the *gsh1* gene, was substituted with arginine, and the amino acid at position 653 (glycine) was substituted with methionine.

The above strain was named CC02-2816 and deposited at the Korean Culture Center of Microorganisms (KCCM) under the Budapest Treaty on December 8, 2020, with Accession No. KCCM12891P.

### Example 2: Additional Improvement Experiment of Strain

In order to further improve the glutathione producing ability of the CC02-2816 strain of Example 1, a mutation was induced in the following manner.

The CC02-2816 strain was cultured on a solid medium and then inoculated into the broth to obtain a culture solution, and the cells were irradiated with UV light using a UV lamp. Thereafter, only the mutant strains that formed colonies by smearing the UV-irradiated culture solution on a plate medium were obtained by isolation, and the nucleotide sequences were analyzed.

As a result of the experiment, it was confirmed that the amino acid at position 37 (glycine) of SOD1 (SEQ ID NO: 1), which is a protein encoded by *sod1* encoding the superoxide dismutase 1 of the strain having improved glutathione content by 26%, was substituted with arginine.

### Example 3: Experiment of 37^{th} Residue Mutation of SOD1 Protein

From the results of Example 2, it was determined that the 37^{th} position of the SOD1 protein would be important for glutathione production, and the wild-type S. *cerevisiae* CEN.PK2-1D and CC02-2816 strain of Example 1 were constructed to express the mutated protein, in which the 37^{th} amino acid of the SOD1 protein was substituted with a different amino acid, thereby determining whether glutathione production was increased.

In order to prepare a strain in which the 37^{th} amino acid of SOD1 protein from S. *cerevisiae* yeast was substituted with arginine, pWAL100 and pWBR100 plasmids were used with reference to the contents disclosed in the literature Lee T. H. et al. (J. Microbiol. Biotechnol. (2006), 16(6), 979-982).

Specifically, PCR was performed as follows using the genomic DNA of the CJ-5 strain as a template. PCR is performed using the primers of SEQ ID NO: 4 and SEQ ID NO: 6 to obtain a partial sequence of the SOD1 N-terminus including the N-terminal BamHI flanking sequence, the initiation codon of the SOD1 ORF, and the G37R mutation coding sequence, and to obtain a partial sequence of SOD1 C-terminus including the C-terminal Xhol flanking sequence, SOD1 ORF termination codon and G37R mutation coding sequence using the primers of SEQ ID NO: 5 and SEQ ID NO: 7. Thereafter, as a result of performing overlap PCR using SEQ ID NO: 4 and SEQ ID NO: 7 based on these two sequences as a template, SOD1 ORF fragments including the SOD1 mutant protein coding sequence, in which the 37^{th} amino acid was substituted with arginine, N-terminal BamHI, and C-terminal Xhol restriction enzymes were obtained. The ORF fragments were cloned into the pWAL100 vector treated with the same enzyme after treatment with BamHI and Xhol, and a pWAL100-SOD1(G37R)) vector was prepared.

Additionally, PCR was performed using SEQ ID NO: 8 and SEQ ID NO: 9 based on the genomic DNA of CJ-5 strain as a template to obtain a 500 bp fragment downstream of the SOD1 ORF termination codon including the N-terminal Spel and C-terminal Ncol restriction enzyme sequences, and the fragment was treated with Spel and Ncol restriction enzymes. After cloning the fragment in pWBR100 treated with the same restriction enzyme, a pWBR100-SOD1 vector was prepared.

In order to prepare a DNA fragment to ultimately be introduced into yeast, a PCR product containing an arginine mutation coding sequence and a part of KIURA3 was obtained based on the previously prepared pWAL100-SOD1 (G37R) vector as a template using the primers of SEQ ID NO: 4 and SEQ ID NO: 10, and a PCR product containing a part of KIURA3 and a 500 bp fragment downstream of the SOD1 termination codon was obtained based on the pWBR100-SOD1 vector as a template using the primers of SEQ ID NO: 11 and SEQ ID NO: 9. Each PCR product was transformed into S. *cerevisiae* CEN.PK2-1D and *S*. *cerevisiae* CC02-2816 at the same molar ratio. PCR was performed under the conditions of denaturation at 95°C for 5 minutes, annealing at 53°C for 1 minute, and polymerization at 72°C for 1 minute per 1 kb. For the transformation of yeast, a lithium acetate method modified from the literature by Geitzs (Nucleic Acid Research, 20(6), 1425) was used. Specifically, after washing the yeast cells with an O.D. of 0.7 to 1.2 twice with lithium acetate/TE buffer, the PCR products and single stranded DNA (Sigma D-7656) were mixed together and subjected to stationary culture in lithium acetate/TE/40% PEG buffer at 30°C for 30 minutes and at 42°C for 15 minutes. Thereafter, the cells were cultured on an SC (2% glucose) agar plate without containing uracil until colonies were observed to obtain a strain into which the SOD1 G37R mutation coding sequence and KIURA3 gene were introduced. Subsequently, in order to remove KIURA3, each strain was cultured in 2 mL of YPD overnight, diluted by 1/100, and plated on an SC (2% glucose) agar plate containing 0.1% 5-FOA to construct a S. *cerevisiae* CEN.PK2-1D SOD1 G37R mutant strain and a *S*. *cerevisiae* CC02-2816 SOD G37R mutant strain from which the uracil marker has been removed. Strains capable of expressing the SOD1 mutant protein substituted with an amino acid other than arginine were also prepared in the same manner except that a primer pair, in which the 37^{th} arginine coding sequence on the primer sequences of SEQ ID NO: 5 and SEQ ID NO: 6 was substituted with a sequence encoding a different amino acid, was used.

**[Table 1]**

| Primers | 5' -> 3' Sequence |
|---|---|
| F_BamHI_SOD1 (SEQ ID NO: 4) | GGTAGGATCC ATGGTTCAAGCAGTCGCAGTGTTAA |
| F_SOD1_G37R (SEQ ID NO: 5) | TTACGAGATCGCT**CGC**AACAGTCCT |
| R_SOD1_G37R (SEQ ID NO: 6) | AGGACTGTT**GCG**AGCGATCTCGTAA |
| R_XhoI_SOD1 (SEQ ID NO: 7) | ATGACTCGAG TTAGTTGGTTAGACCAATGACACCA |
| F_Spel_SOD1_DW (SEQ ID NO: 8) | TAGAACTAGT TGTTAATGATAATATACTTGAATA |
| R_Ncol_SOD1_DW (SEQ ID NO: 9) | GCTGCCATGG ACACTACTAGCTCCTGAAGACCAAG |
| R_AL killer (SEQ ID NO: 10) | GAGCAATGAACCCAATAACGAAATCTT |
| F_BR killer (SEQ ID NO: 11) | CTTGACGTTCGTTCGACTGATGAG |

The results of measuring the concentration and content of glutathione (GSH) produced by culturing each strain prepared above for 26 hours are shown in Tables 2 and 3, and Figs. 1 and 2 below.

**[Table 2]**

| ***S*. *cerevisiae* CC02-2816** | | |
|---|---|---|
| | Content (fold) | Concentration (fold) |
| **WT (Wild-Type)** | **1.00** | **1.00** |
| SOD1 G37A | 1.25 | 1.16 |
| SOD1 G37C | 1.26 | 1.14 |
| SOD1 G37D | 1.25 | 1.11 |
| SOD1 G37E | 1.24 | 1.14 |
| SOD1 G37F | 1.18 | 0.90 |
| SOD1 G37H | 1.23 | 1.12 |
| SOD1 G37I | 1.31 | 1.16 |
| SOD1 G37K | 1.06 | 1.14 |
| SOD1 G37L | 1.28 | 1.17 |
| SOD1 G37M | 1.18 | 0.95 |
| SOD1 G37N | 1.28 | 1.14 |
| SOD1 G37P | 1.13 | 0.73 |
| SOD1 G37Q | 1.24 | 1.18 |
| SOD1 G37R | 1.26 | 1.18 |
| SOD1 G37S | 1.25 | 1.24 |
| SOD1 G37T | 1.22 | 1.22 |
| SOD1 G37V | 1.21 | 1.24 |
| SOD1 G37W | 1.16 | 0.69 |
| SOD1 G37Y | 1.21 | 1.21 |

**[Table 3]**

| ***S*. *cerevisiae* CEN.PK2-1D** | | |
|---|---|---|
| | Concentration (fold) | Content (fold) |
| **CEN.PK** | **1.00** | **1.00** |
| SOD1 G37A | 1.06 | 1.10 |
| SOD1 G37C | 1.05 | 1.08 |
| SOD1 G37D | 1.08 | 1.11 |
| SOD1 G37E | 1.07 | 1.12 |
| SOD1 G37F | 1.34 | 1.26 |
| SOD1 G37H | 1.24 | 1.11 |
| SOD1 G37I | 1.32 | 1.19 |
| SOD1 G37K | 1.19 | 1.13 |
| SOD1 G37L | 1.31 | 1.25 |
| SOD1 G37M | 1.18 | 1.07 |
| SOD1 G37N | 1.27 | 1.17 |
| SOD1 G37P | 1.12 | 1.12 |
| SOD1 G37Q | 1.26 | 1.17 |
| SOD1 G37R | 1.10 | 1.06 |
| SOD1 G37S | 1.26 | 1.21 |
| SOD1 G37T | 1.22 | 1.18 |
| SOD1 G37V | 1.18 | 1.12 |
| SOD1 G37W | 1.26 | 1.23 |
| SOD1 G37Y | 1.23 | 1.14 |

Based on the results, it was confirmed that the glutathione producing ability can be increased by substituting the amino acid at position 37 with different amino acids, and accordingly, it can be implied that the SOD1 protein variant provided in the present application can be effectively used for the production of gamma-glutamyl peptides.

From the foregoing, a skilled person in the art to which the present application pertains will be able to understand that the present application may be embodied in other specific forms without modifying the technical concepts or essential characteristics of the present application. In this regard, the exemplary embodiments disclosed herein are only for illustrative purposes and should not be construed as limiting the scope of the present application. The scope of the present application is therefore indicated by the appended claims rather than by the foregoing description. All changes which come within the meaning and range of equivalency of the claims are to be embraced within the scope of the present application.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Saccharomyces cerevisiae CC02-2816 | Accession number given by the
INTERNATIONAL DEPOSITARY AUTHORITY:
KCCM12891P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on December. 08. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 08. 2020.

¹ Where Rule 6.4(d) applies, such date is the date on which the status of international depositary authority was
acquired.

Form BP/4 (sole page)

한국미생물보존센터 [?]
03641 서울시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A superoxide dismutase 1 (SOD1) variant, in which an amino acid corresponding to the position 37 from the N-terminus of the amino acid sequence of SEQ ID NO: 1 is substituted with a different amino acid.

2. The variant of claim 1, wherein the different amino acid is selected from alanine, valine, leucine, isoleucine, methionine, phenylalanine, tryptophan, proline, serine, threonine, cysteine, tyrosine, asparagine, glutamine, aspartic acid, glutamic acid, lysine, arginine, and histidine.

3. The variant of claim 1, wherein the variant comprises any one of amino acid sequences of SEQ ID NOS: 12 to 30.

4. A polynucleotide encoding the superoxide dismutase 1 variant of any one of claims 1 to 3.

5. A microorganism, comprising any one or more of the superoxide dismutase 1 variant of any one of claims 1 to 3; and a polynucleotide encoding the variant.

6. The microorganism of claim 5, wherein the microorganism produces glutathione or derivatives thereof.

7. The microorganism of claim 5, wherein the microorganism belongs to the genus *Saccharomyces.*

8. The microorganism of claim 7, wherein the microorganism is *Saccharomyces cerevisiae.*

9. A method for producing glutathione or derivatives thereof, comprising: culturing a microorganism comprising any one or more of the superoxide dismutase 1 variant of any one of claims 1 to 3; and a polynucleotide encoding the variant in a medium.

10. The method of claim 9, further comprising recovering glutathione or derivatives thereof from at least one material selected from the cultured microorganism, a dried product thereof, an extract thereof, a culture product thereof, and a lysate thereof.
